# FASCICULE DE BREVET EUROPEEN

(11) **EP 2 753 245 B1**
(45) Date de publication et mention de la délivrance du brevet: **03.01.2018**
(21) Numéro de dépôt: 12756449.0
(22) Date de dépôt: 05.09.2012
(51) Int. Cl.: A61B 10/02, A61B 17/3205

(54) **DISPOSITIF POUR LE PRELEVEMENT LAPAROSCOPIQUE D'UN ECHANTILLON CYLINDRIQUE SUPERFICIEL D'UN TISSU DU CORPS HUMAIN OU ANIMAL**
VORRICHTUNG ZUR LAPAROSKOPISCHEN ENTFERNUNG EINER ZYLINDRISCHEN PROBE EINES MENSCHLICHEN ODER TIERISCHEN KÖRPERGEWEBES
DEVICE FOR THE LAPAROSCOPIC REMOVAL OF A SUPERFICIAL CYLINDRICAL SAMPLE OF A HUMAN OR ANIMAL BODY TISSUE

(30) Priorité: 05.09.2011 FR 1157854
(43) Date de publication de la demande: 16.07.2014
(73) Titulaire: Endodiag, 91058 Evry Cedex (FR)
(72) Inventeur: REAL, Cécile, 75018 Paris (FR); HENRI, Patrick, 92270 Bois Colombes (FR)
(74) Mandataire: Regimbeau
(86) Numéro de dépôt international: PCT/EP2012/067268
(87) Numéro de publication internationale: WO 2013/034573

(56) Documents cités:
- WO-A1-02/065919
- WO-A2-2009/079155
- DE-U1-202005 016 761
- US-A- 4 461 305
- US-A- 5 353 804

## Description

### DOMAINE DE L'INVENTION

La présente invention concerne un dispositif pour le prélèvement laparoscopique d'un échantillon cylindrique superficiel d'un tissu du corps humain ou animal.

### ARRIERE PLAN DE L'INVENTION

La biopsie est une procédure répandue à des fins de diagnostic.

En effet, un échantillon prélevé dans un tissu du corps humain ou animal peut être analysé en laboratoire en vue de détecter d'éventuelles lésions, maladies, etc.

Afin de limiter les effets post-opératoires sur le patient, des procédures laparoscopiques, selon lesquelles on introduit un instrument de prélèvement dans le corps à travers une incision de petite taille, ont été développées.

Parmi les tissus dans lesquels une biopsie est susceptible d'être pratiquée, on distingue notamment les organes pleins, assimilables à des volumes, et les membranes, dont la surface est grande par rapport à l'épaisseur.

Ainsi, par exemple, la biopsie pour le diagnostic de l'endométriose s'effectue dans le péritoine, qui est une membrane relativement peu épaisse (quelques millimètres) entourant les viscères.

Dans un tel cas, on cherche à prélever un échantillon superficiel du péritoine sans perforer celui-ci, ce qui implique que l'épaisseur de l'échantillon soit inférieure à l'épaisseur du péritoine.

Or, les aiguilles de biopsie connues permettent de prélever, dans un organe plein, tel que le foie par exemple, dans lequel elles pénètrent profondément, une carotte épaisse mais de faible diamètre (par exemple, un cylindre de l'ordre de 1 mm de diamètre sur 5 à 6 mm d'épaisseur).

De telles aiguilles ne sont pas adaptées pour un prélèvement sur le péritoine car elles impliqueraient une perforation de celui-ci, engendrant un traumatisme important pour le patient.

Il existe donc un besoin pour un dispositif de prélèvement laparoscopique d'un échantillon d'un tissu interne au corps humain ou animal, notamment lorsque ledit tissu est une membrane corporelle ou un organe peu épais.

Le document US 4,461,305 concerne un dispositif de prélèvement comprenant un tube extérieur solidaire d'une poignée de préhension, et un tube intérieur mobile en rotation par rapport à la poignée. Le tube intérieur présente une arête tranchante à son extrémité distale et supporte, à cette même extrémité, un fil tranchant pivotable entre une position escamotée où l'arête tranchante du fil est dans le prolongement de la paroi du tube et une position de coupe où l'arête tranchante du fil est dirigée vers l'intérieur du tube. L'arête tranchante du tube intérieur permet de découper la paroi périphérique de l'échantillon lorsque le tube pénètre dans le tissu. Le pivotement du fil tranchant par rapport au tube intérieur permet ensuite de détacher l'échantillon du tissu par découpe de sa face située dans la profondeur du tissu.

Un but de l'invention est donc de procurer un dispositif utilisable en laparoscopie pour prélever un échantillon superficiel d'un tissu, par exemple d'une membrane, sans perforer ledit tissu.

### BREVE DESCRIPTION DE L'INVENTION

Conformément à l'invention, il est proposé un dispositif pour le prélèvement laparoscopique d'un échantillon cylindrique superficiel d'un tissu du corps humain ou animal, comprenant :
- un tube dont le diamètre intérieur détermine le diamètre de l'échantillon à prélever, et présentant une arête tranchante à son extrémité distale,
- une poignée de préhension, solidaire du tube dans la région d'une extrémité proximale dudit tube,
- un couteau agencé à l'intérieur du tube et présentant une arête tranchante,
- un dispositif d'actionnement du couteau, comprenant un élément d'actionnement connecté à la poignée de préhension de manière à donner au couteau un mouvement hélicoïdal dans la direction d'une extrémité distale du tube puis un mouvement de rotation pour détacher l'échantillon du tissu.

De manière particulièrement avantageuse, l'élément d'actionnement est en outre agencé de sorte à, après ledit mouvement de rotation et sous l'effet d'une pression exercée dans la direction de l'extrémité distale, faire coulisser le couteau dans le tube de sorte à le faire dépasser de l'extrémité distale du tube en vue de libérer l'échantillon prélevé.

Selon une forme d'exécution avantageuse de l'invention, ledit dispositif d'actionnement comprend :
- un élément de connexion solidaire du tube,
- une tige d'actionnement solidaire, à son extrémité distale, du couteau,
- un élément de retenue solidaire de la tige d'actionnement, relié à l'élément de connexion par une liaison hélicoïdale dans une position proximale de la tige d'actionnement par rapport au tube et par une liaison pivot dans une position distale de la tige d'actionnement.

Selon un mode particulier de réalisation de l'invention, ledit élément de connexion comprend une portion proximale taraudée coopérant avec une collerette filetée de l'élément de retenue pour former ladite liaison hélicoïdale et un alésage distal plus large que ladite collerette pour former ladite liaison pivot.

Par ailleurs, ledit dispositif d'actionnement peut comprendre une molette solidaire de l'extrémité distale de la tige d'actionnement.

Selon une forme particulière d'exécution de l'invention, l'élément de retenue est maintenu dans l'élément de connexion par une bague, ladite bague comprenant une butée proximale pour l'élément de retenue vis-à-vis de l'élément de connexion, ladite butée étant escamotable pour libérer l'élément de retenue de l'élément de connexion.

Par exemple, le diamètre intérieur du tube est compris entre 2 et 5 mm.

La course longitudinale du couteau dans son mouvement hélicoïdal peut quant à elle être comprise entre 1 et 5 mm.

Selon un mode de réalisation préféré de l'invention, la poignée de préhension a une forme générale de pistolet, l'élément d'actionnement comprenant une gâchette.

Ladite poignée comprend en outre avantageusement un bouton de sécurité mobile entre une position de blocage et une position de libération de la gâchette.

### BREVE DESCRIPTION DES DESSINS

D'autres caractéristiques et avantages de l'invention ressortiront de la description détaillée qui va suivre, en référence aux dessins annexés sur lesquels :
- la figure 1 présente des vues de côté et en coupe d'un mode de réalisation préféré du dispositif d'actionnement selon l'invention, dans une position initiale de prélèvement,
- la figure 2 présente des vues de côté, de face, de dessus et en coupe d'un mode de réalisation préféré de l'invention,
- la figure 3 présente des vues de côté et en coupe d'un deuxième mode de réalisation de l'invention,
- les figures 4A à 4E illustrent différentes étapes de l'utilisation du dispositif pour effectuer un prélèvement.

### DESCRIPTION DETAILLEE DE L'INVENTION

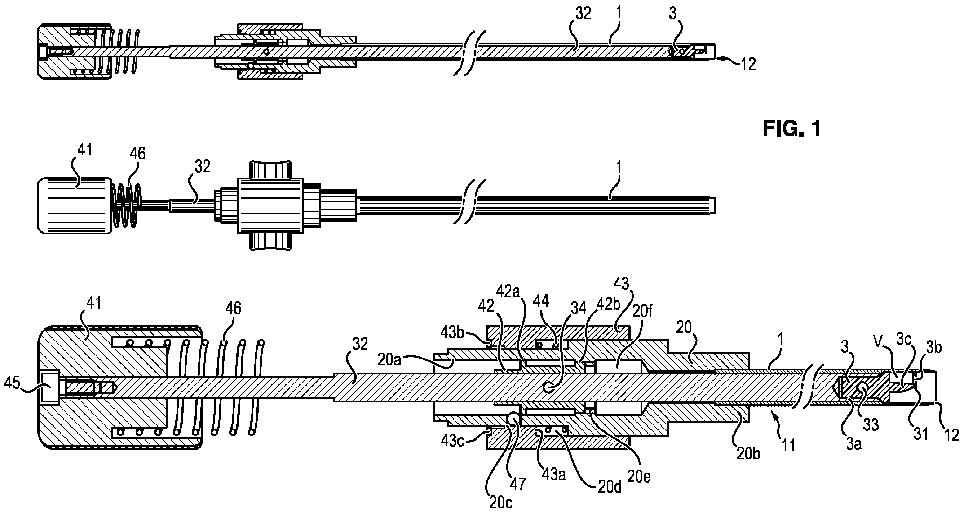

La figure 1 présente une vue de côté et une vue en coupe du dispositif d'actionnement, avec des détails A et B respectivement de la partie distale et de la partie proximale du dispositif.

Dans le présent texte, on qualifie de « distal » le côté le plus proche du tissu dans lequel l'échantillon doit être prélevé, et de « proximal » le côté opposé, le plus proche de la main de l'utilisateur.

Le tube 1 est un tube dont le diamètre intérieur est égal au diamètre de l'échantillon que l'on doit prélever.

Par exemple, le diamètre intérieur du tube 1 peut être compris entre 2 et 5 mm.

La longueur du tube peut dépendre de l'emplacement du tissu à prélever dans le corps humain ou animal.

Typiquement, la longueur du tube 1 peut être de l'ordre de 300 mm.

L'extrémité distale 10 du tube 1 présente une arête tranchante 12, similaire à celle d'un emporte-pièce.

Le tube 1 est ainsi adapté pour pénétrer dans le tissu en découpant la paroi périphérique de l'échantillon sous l'effet d'une pression exercée par un utilisateur.

A l'intérieur du tube 1 est agencé un couteau 3, qui, lorsqu'il est dans une position appropriée par rapport au tube 1, permet de détacher le fond de l'échantillon, c'est-à-dire le disque situé dans la profondeur du tissu.

A cet effet, le couteau 3 présente une arête tranchante 31.

De préférence, ladite arête tranchante 31 est perpendiculaire à la paroi du tube 1, de sorte qu'un mouvement de rotation du couteau 3 permette de découper l'échantillon dans une direction perpendiculaire à sa paroi cylindrique.

Le couteau 3 présente avantageusement une portion proximale 3a de forme générale cylindrique destinée à être solidarisée à une tige d'actionnement 32 (décrite plus bas) et une portion distale 3b en forme d'arc de cercle portant l'arête tranchante 31.

L'arête tranchante 31 est de préférence rectiligne, selon un diamètre de la portion distale en arc de cercle 3b. De manière alternative, l'arête tranchante peut être courbe ou présenter tout autre profil approprié.

Les portions 3a et 3b présentent un diamètre extérieur sensiblement égal au diamètre intérieur du tube 1.

Lesdites portions 3a et 3b sont reliées par un pied 3c dont le diamètre est inférieur au diamètre intérieur du tube 1.

Comme on le verra plus bas, le volume V compris entre la paroi intérieure du tube 1, la face distale de la portion 3a et la face proximale de la portion 3b du couteau définit le volume de l'échantillon à prélever.

Le couteau 3 est rendu solidaire d'une tige d'actionnement 32 par sa portion proximale 3a.

Par exemple, comme illustré à la figure 1, la portion proximale 3a du couteau est partiellement insérée dans l'extrémité distale de la tige 32 et fixée dans celle-ci au moyen d'une goupille 33. Naturellement, tout autre moyen de solidarisation peut être employé.

La tige d'actionnement 32 présente un diamètre légèrement inférieur au diamètre intérieur du tube 1 de sorte à pouvoir coulisser en translation et en rotation dans celui-ci sans frottement et à présenter une résistance mécanique adaptée à l'utilisation.

A son extrémité proximale, la tige d'actionnement 32 est rendue solidaire d'une molette 41.

Dans l'exemple illustré ici, la molette 41 est fixée sur la tige d'actionnement 32 au moyen d'une vis 45, mais il va de soi que l'on pourrait employer tout autre mode de fixation sans pour autant sortir du cadre de la présente invention.

Par ailleurs, la tige d'actionnement 32 porte également, dans sa portion proximale, un élément de retenue 42.

Ledit élément de retenue 42 présente une forme générale cylindrique, avec un diamètre intérieur légèrement supérieur à celui de la tige d'actionnement 32 de sorte à pouvoir être enfilé sur celle-ci.

Dans l'exemple illustré ici, l'élément de retenue 42 est rendu solidaire de la tige d'actionnement 32 au moyen d'une goupille 34, mais l'invention n'est bien sûr pas limitée à ce mode de fixation.

Dans sa partie proximale, l'élément de retenue 42 présente une collerette lisse 42a et, dans sa partie distale, une collerette filetée 42b.

Par ailleurs, le dispositif de prélèvement comprend une poignée de préhension 2.

La poignée 2 comprend un élément de connexion 20 qui permet de raccorder, par l'intermédiaire de l'élément de retenue 42, la tige d'actionnement 32 (qui, comme indiqué plus haut, est solidaire de la molette 41 et du couteau 3) et le tube 1.

Plus précisément, ledit élément de connexion 20 présente une forme générale cylindrique avec un passage intérieur présentant un diamètre intérieur supérieur au diamètre extérieur de la tige d'actionnement 32 pour permettre l'insertion et le coulissement de ladite tige 32 dans ledit passage.

De sa partie proximale vers sa partie distale, le passage intérieur comprend successivement un logement 20a dont le diamètre intérieur est supérieur au diamètre extérieur des collerettes 42a et 42b, un alésage taraudé 20a adapté pour coopérer avec le filetage de la collerette 42b, un logement 20f dont le diamètre intérieur est supérieur au diamètre de la collerette 42b.

Dans sa partie distale 20b, l'élément de connexion 20 est solidaire de l'extrémité proximale 11 du tube 1.

Par ailleurs, l'élément de connexion 20 est entouré par une bague 43.

Ladite bague 43 est agencée pour coulisser sur une paroi extérieure de l'élément de connexion 20 dans la direction de l'extrémité distale du dispositif à l'encontre de la réaction d'un élément élastique 44, qui est par exemple un ressort de traction.

Ledit ressort 44 est logé entre un épaulement intérieur 20d de l'élément de connexion 20 et un épaulement intérieur 43a de la bague 43.

Dans sa partie proximale, la bague 43 est maintenue en butée vis-à-vis de l'élément de connexion 20 par un clip 43b.

Dans sa partie entourée par la bague 43, le passage 20a de l'élément de connexion 20 comprend un orifice traversant 20c constituant un logement pour une bille 47.

La bille 47 présente un diamètre supérieur à la profondeur de l'orifice 20c à l'intérieur duquel elle est logée, de sorte que dans une première position du dispositif, dite position initiale de prélèvement, la bille 47 est en saillie vers l'intérieur du passage 20a par rapport à la paroi dudit passage.

Dans cette position, la collerette filetée 42b de l'élément de retenue 42 décrit plus haut est vissée dans un alésage taraudé 20e de l'élément de connexion 20.

L'alésage taraudé 20e présente une longueur supérieure à la longueur de la collerette filetée 42b.

La collerette proximale 42a de l'élément de retenue 42 est située dans la partie distale du passage 20a par rapport à la bille 47, de sorte que la bille 47 constitue une butée dans la direction proximale pour la tige d'actionnement 32.

Lorsque l'on actionne la bague 43 dans la direction distale à l'encontre du ressort 44, un évidement 43c pratiqué dans la paroi intérieure de la bague 43 vient en vis-à-vis de l'orifice traversant 20c contenant la bille 47. L'évidement 43c présente une profondeur suffisante pour que la bille 47 soit alors totalement escamotée dans l'orifice traversant 20c et l'évidement 43c et ne s'oppose donc plus au coulissement de la butée 42 et de la tige d'actionnement 32 dans la direction proximale.

Ainsi, en poussant sur la bague 43 dans la direction distale, on peut dissocier le dispositif de prélèvement en deux sous-ensembles : la tige d'actionnement 32, le couteau 3 et la molette 41 d'une part, et l'élément de connexion 20, le tube 1 et la bague 43 d'autre part.

Comme on le verra plus bas, cette aptitude du dispositif à être démonté est avantageuse puisqu'elle permet de mieux nettoyer et de stériliser le dispositif, ce qui le rend adapté à des usages multiples.

A cet effet, les composants du dispositif mentionnés ci-dessus sont fabriqués en des matériaux compatibles avec la stérilisation.

On peut citer parmi les matériaux préférés : l'inox, le polyéther éther kétone (PEEK), le polyphénylsulfone (Radel®), mais l'homme du métier pourra choisir tout autre matériau remplissant les critères mécaniques et de stérilisabilité requis.

Cependant, le dispositif peut également être destiné à un usage unique et les contraintes de démontage, de remontage et de stérilisation ne sont alors pas à prendre en compte dans la conception dudit dispositif.

Par conséquent, tout en conservant le mode de prélèvement de l'échantillon décrit ci-dessus (c'est-à-dire avec un mouvement hélicoïdal suivi d'un mouvement de rotation du couteau), la conception du dispositif et les matériaux employés pourront différer de ceux des exemples décrits afin de procurer un dispositif à usage unique optimal du point de vue de coût, de ses conditions d'utilisation, etc.

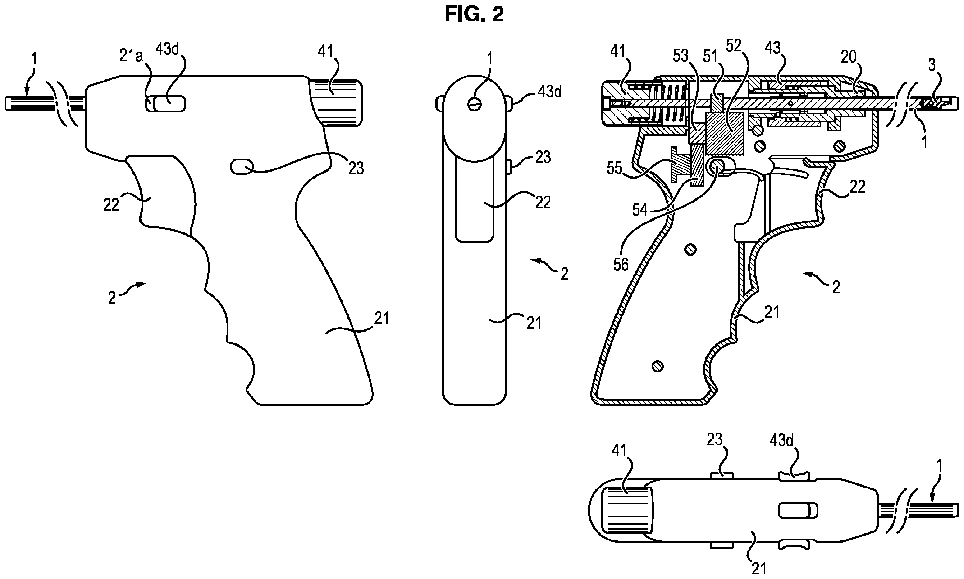

Selon une première forme d'exécution de l'invention, illustrée à la figure 2, la poignée de préhension 2 se présente sous la forme d'un pistolet muni d'une gâchette pour l'actionnement du couteau.

Cette conception du dispositif est particulièrement avantageuse car la poignée présente une forme ergonomique similaire à celle d'autres outils chirurgicaux, de sorte que l'utilisateur utilisera ce nouveau dispositif de manière intuitive.

Cette forme particulière n'est naturellement pas limitative et l'on pourra définir tout autre forme ergonomique pour la poignée sans pour autant sortir du cadre de la présente invention.

La poignée comprend un boîtier 21 présentant une forme générale de pistolet comprenant une zone de préhension adaptée à la prise en main par un utilisateur.

Le boîtier peut être formé de deux coques sensiblement symétriques qui peuvent être assemblées de manière démontable (par exemple au moyen de vis) pour permettre d'accéder au dispositif d'actionnement en vue d'une éventuelle réparation.

Le dispositif d'actionnement décrit plus haut est assemblé à l'intérieur dudit boîtier.

De préférence, l'élément de connexion 20 est maintenu dans un logement approprié à l'intérieur du boîtier, deux orifices de passage étant prévus sur le boîtier pour le tube 1 d'une part et pour la molette 41 d'autre part.

Par ailleurs, un élément élastique 46, par exemple un ressort de traction, est interposé entre le boîtier et la molette 41 de sorte à exercer une réaction dans la direction distale sur la molette.

Par ailleurs, la bague 43 est munie sur au moins un de ses côtés d'un bouton d'actionnement 43d. Ledit bouton 43d est en saillie par rapport à la paroi extérieure du boîtier 21 à travers un trou oblong 21a. Ceci permet à l'utilisateur d'actionner la bague 43 bien que celle-ci soit enfermée à l'intérieur du boîtier 21.

La poignée comprend également une gâchette 22 en appui sur un élément élastique 56, qui est reliée à la tige d'actionnement par une poulie dentée 55 et une série d'engrenages comprenant des roues dentées 51, 52, 53 et 54.

En particulier, la roue dentée 51 est solidaire de la tige d'actionnement 32 de sorte qu'une pression exercée sur la gâchette est transformée en un mouvement de rotation de la tige d'actionnement.

La course d'enfoncement de la gâchette 22 est définie de telle sorte qu'une première partie de la course corresponde au vissage de la collerette 42b dans l'alésage 20e et une seconde partie de la course corresponde à la rotation de la collerette 42b dans le logement 20f.

Ainsi, une seule pression sur la gâchette 22 permet le détachement de l'échantillon du tissu.

De manière avantageuse, la poignée comprend en outre un bouton de sécurité 23 qui est mobile entre une position dans laquelle il bloque l'actionnement de la gâchette (ce qui évite tout déclenchement intempestif) et une position dans laquelle il la libère.

Pour actionner la gâchette, l'utilisateur doit donc simultanément enfoncer ledit bouton de sécurité 23.

Une fois la gâchette enfoncée, elle reste dans cette position et une pression exercée sur le bouton de sécurité 23, combinée à l'effort de rappel de l'élément élastique 56, permet de la ramener à sa position initiale.

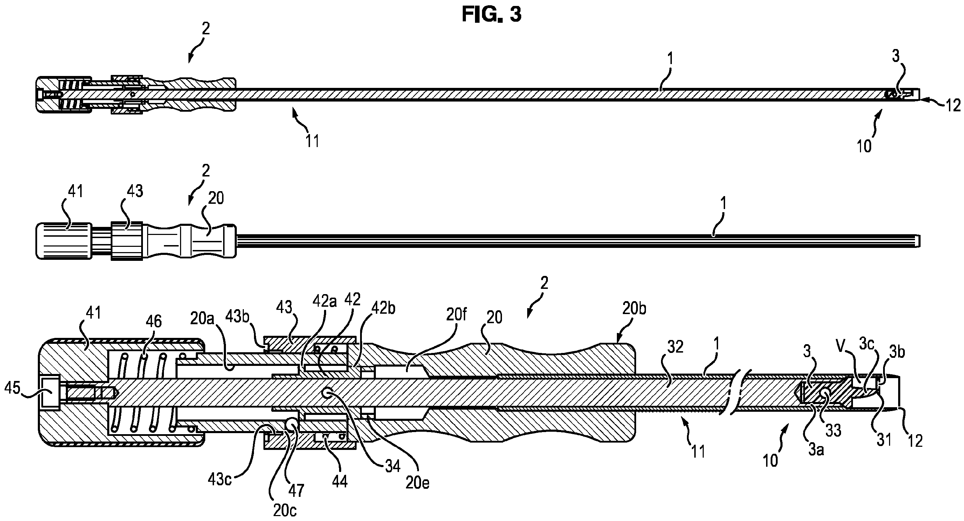

Selon une deuxième forme d'exécution du dispositif, illustrée à la figure 3, la poignée de préhension 2 présente une forme tubulaire comprenant une première partie solidaire du tube 1 et une deuxième partie agencée en rotation et en coulissement par rapport à la première partie et solidaire de la tige d'actionnement 32.

Ladite première partie comprend l'élément de connexion 20 décrit plus haut qui, dans ce mode de réalisation, remplit donc à la fois la fonction de connexion du tube 1 et de la tige d'actionnement 32 et la fonction de préhension par un utilisateur.

A cet effet, la paroi externe de l'élément de connexion 32 est avantageusement pourvue de formes adaptées à la prise en main par un utilisateur.

Ladite deuxième partie comprend le sous-ensemble constitué par la molette 41, la tige d'actionnement 32 et le couteau 3 décrit plus haut.

Dans ce mode de réalisation, un élément élastique 46 (par exemple un ressort de traction) est interposé entre la molette 41 et l'élément de connexion 20.

Dans la position initiale de prélèvement mentionné ci-dessus, l'élément élastique 46 est à l'état libre ou peu contraint.

L'élément de retenue 42 étant en butée proximale contre la bille 47, une rotation de la molette 41 dans le sens antihoraire est impossible.

Il est en revanche possible de tourner la molette 41 dans le sens horaire.

Tant que la collerette filetée 42b de l'élément de retenue 42 est en prise avec l'alésage taraudé 20e du logement 20, le mouvement de la tige d'actionnement 32 (et donc du couteau 3) est un mouvement hélicoïdal dans la direction distale.

Une fois que la collerette filetée 42b de l'élément de retenue 42 n'est plus en prise avec l'alésage taraudé 20e de l'élément de connexion 20, ladite collerette filetée 42b se trouve dans le logement 20f.

Dans cette position, dite position de détachement de l'échantillon, une rotation de la molette 41 dans le sens horaire conduit à une rotation pure de la tige d'actionnement 32 (et donc du couteau 3), c'est-à-dire une rotation sans déplacement dans la direction distale.

Enfin, à partir de cette position de détachement, il est également possible d'exercer une pression sur la molette 41 dans la direction distale à l'encontre de l'élément élastique 46.

La longueur du logement 20f définit alors une course correspondant à la course selon laquelle le couteau 3 est extrait du tube 1.

Cette position dans laquelle le couteau dépasse de l'extrémité distale 10 du tube 1 est dite position de libération de l'échantillon.

La procédure d'utilisation du dispositif est la suivante.

Sur les figures qui illustrent cette procédure, c'est le premier mode de réalisation du dispositif (c'est-à-dire celui dans lequel la poignée de préhension est le pistolet décrit ci-dessus et illustré à la figure 2) qui est représenté.

Cependant, sauf mention contraire, la procédure est identique pour les autres formes d'exécution du dispositif.

Avant de procéder au prélèvement, l'utilisateur vérifie que le dispositif est dans la position initiale de prélèvement, c'est-à-dire que le couteau 3 (par l'intermédiaire de la tige d'actionnement 32 et de l'élément de retenue 42) est en butée proximale dans le tube 1.

A cet effet, l'utilisateur tente de tourner la molette 41 dans le sens antihoraire.

Si la rotation est impossible, le dispositif est dans la position initiale souhaitée.

Sinon, l'utilisateur tourne la molette 41 dans le sens antihoraire jusqu'à arriver en butée.

La découpe de l'échantillon est effectuée en deux étapes : une première étape de découpe de la paroi cylindrique de l'échantillon, suivie d'une étape de détachement de l'échantillon du tissu par découpe de la face de l'échantillon située en profondeur dans le tissu.

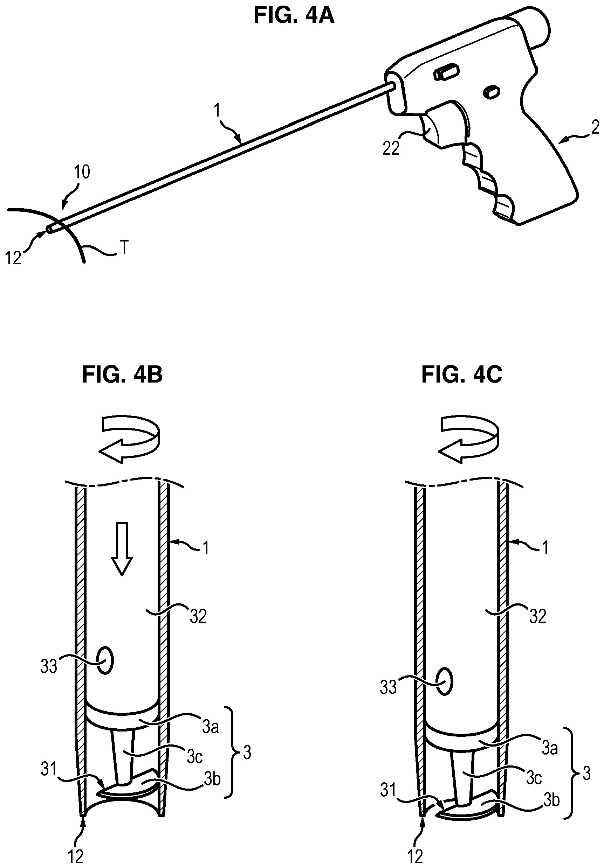

Dans la première étape de découpe, illustrée à la figure 4A, l'utilisateur introduit le tube 1 à travers une canule (non représentée ici) et enfonce la partie distale 10 du tube 1 dans le tissu T dans lequel il souhaite prélever l'échantillon.

Le tube 1 remplit la fonction d'un emporte-pièce en découpant la paroi cylindrique au moyen de son arête tranchante 12.

La profondeur selon laquelle le tube 1 est enfoncé dépend de la nature du tissu dans lequel on effectue le prélèvement ; elle est typiquement comprise entre 1 et 5 mm.

Une fois la profondeur souhaitée atteinte, l'utilisateur actionne le dispositif d'actionnement en vue de détacher l'échantillon.

Dans le cas où la poignée de préhension est un pistolet tel qu'illustré à la figure 2, l'utilisateur appuie sur la gâchette 22.

Dans le cas où la poignée de préhension est une poignée cylindrique telle qu'illustrée à la figure 3, l'utilisateur maintient l'élément de connexion 20 dans la paume de sa main et tourne la molette 41 dans le sens horaire en le tenant entre le pouce et l'index.

Naturellement, on pourra définir toute autre forme de poignée de préhension et concevoir à cet effet tout autre moyen approprié pour mettre en oeuvre le dispositif d'actionnement sans pour autant sortir du cadre de la présente invention.

Dans tous les cas, cette action de l'utilisateur a pour effet de faire avancer le couteau 3 dans le tube 1 selon un mouvement hélicoïdal (figure 4B). Ceci correspond au vissage de la collerette filetée 42b de l'élément de retenue 42 dans l'alésage taraudé 20e de l'élément de connexion 20.

Après quelques tours (par exemple deux ou trois), l'avancement du couteau 3 est stoppé car la collerette filetée 42b n'est plus en prise avec l'alésage taraudé 20e et se trouve dans le logement 20f.

Lorsque l'utilisateur continue de tourner la molette 41, le couteau 3 est alors uniquement animé d'un mouvement de rotation qui permet de découper le fond de l'échantillon (figure 4C).

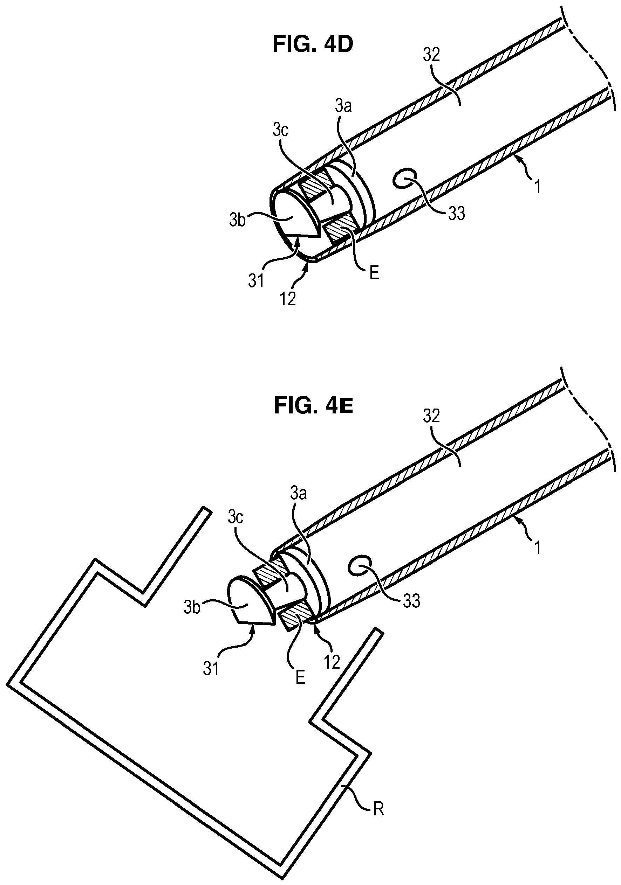

Comme on peut le voir à la figure 4D, l'échantillon E est alors emprisonné dans le tube 1 et maintenu à l'intérieur du tube 1 par le couteau 3, ce qui permet d'assurer que l'échantillon ne tombe pas du dispositif lors du retrait de celui-ci hors du corps du patient.

L'utilisateur retire le dispositif de la canule et introduit l'extrémité distale du tube 1 dans un récipient R destiné au transport de l'échantillon vers le lieu d'analyse.

Il exerce alors une pression sur la molette 41 pour extraire le couteau 3 du tube 1 (figure 4E).

L'échantillon se libère et tombe dans le récipient, par gravité et/ou à l'aide d'un outil approprié (aiguille, pince, etc.).

Le cas échéant, l'utilisateur peut effectuer un ou plusieurs autres prélèvements dans le même tissu.

A cet effet, il doit remettre le dispositif dans la position initiale mentionnée plus haut et réitérer les étapes décrites ci-dessus.

A partir de la position de libération de l'échantillon, ceci implique de relâcher la pression exercée sur la molette 41 qui, sous l'effet de la réaction de l'élément élastique 46, reprend sa position proximale libre.

Dans le mode de réalisation dans lequel la poignée de préhension est le pistolet de la figure 2, l'utilisateur doit en outre appuyer sur le bouton de sécurité, ce qui ramène la gâchette 22 dans une position saillante et l'élément de retenue 42 en butée contre la bille 47.

Dans le mode de réalisation dans lequel la poignée de préhension est la poignée cylindrique de la figure 3, l'utilisateur tourne alors la molette 41 dans le sens antihoraire, ce qui se traduit par un vissage de la collerette 42b de l'élément de retenue 42 dans l'alésage fileté 20e de l'élément de connexion 20, dans la direction proximale. Dans le même temps, le couteau 3 rentre dans le tube 1. Lorsque la collerette proximale 42a de l'élément de retenue 42 arrive en butée contre la bille 47, l'utilisateur ne peut plus tourner la molette 41 dans le sens antihoraire et sait que le dispositif est dans la position initiale de prélèvement.

Après utilisation, le dispositif peut être nettoyé et stérilisé s'il est conçu de sorte à être réutilisable, ou bien jeté s'il est à usage unique.

Le cas échéant, pour faciliter le nettoyage, le dispositif est démonté selon les deux sous-ensembles mentionnés plus haut.

La procédure de démontage d'un dispositif réutilisable est la suivante.

L'utilisateur déplace la bague 43 dans la direction distale (le cas échéant, à l'aide du bouton d'actionnement 43d), ce qui conduit à l'escamotage de la bille 47 dans l'orifice traversant 20c et l'évidement 43c.

L'utilisateur tourne alors la molette 41 dans le sens antihoraire pour dévisser la collerette 42b de l'élément de retenue 42 de l'alésage taraudé 20e de l'élément de connexion 20.

Une fois que la collerette 42b n'est plus en prise avec l'alésage 20e, l'utilisateur peut alors retirer de l'élément de connexion 20 le sous-ensemble constitué de la molette 41, de la tige d'actionnement 32, de l'élément de retenue 42 et du couteau 43.

L'autre sous-ensemble est constitué de l'élément de connexion 20, du tube 1 et de la bague 43.

De préférence, l'élément élastique 46 est solidaire de l'élément de connexion 20 ou de la molette 41, ce qui évite tout risque de perte et facilite la manipulation de pièces éparses.

Les deux sous-ensembles peuvent alors être nettoyés et stérilisés.

La procédure de remontage du dispositif réutilisable est la suivante.

L'utilisateur saisit l'élément de connexion 20 et déplace la bague 43 dans la direction distale.

Il peut alors insérer le sous-ensemble constitué de la molette 41, de la tige d'actionnement 32, de l'élément de retenue 42 et du couteau 43 dans l'élément de connexion 20.

Il tourne la molette 41 dans le sens horaire pour visser la collerette 42b de l'élément de retenue 42 dans l'alésage taraudé 20e de l'élément de connexion.

Ensuite, il relâche la bague 43 qui, sous la réaction de l'élément élastique 44, reprend sa position proximale libre contre le clip 43. Dans le même temps, la bille 47 est chassée de l'évidement 43c et redevient saillante vers l'intérieur par rapport à la paroi du passage 20a.

Il va de soi que le mouvement hélicoïdal puis rotatif du couteau pourra être procuré par d'autres éléments sans pour autant sortir de la portée de la présente invention.

## Revendications

1. Dispositif pour le prélèvement laparoscopique d'un échantillon cylindrique superficiel d'un tissu du corps humain ou animal, comprenant:
- un tube (1) dont le diamètre intérieur détermine le diamètre de l'échantillon à prélever, et présentant une arête tranchante (12) à son extrémité distale (10), ladite arête étant apte à découper la paroi périphérique de l'échantillon lorsque le tube (1) pénètre dans le tissu,
- une poignée de préhension (2), solidaire du tube (1) dans la région d'une extrémité proximale (11) dudit tube (1),
- un couteau (3) agencé à l'intérieur du tube (1) et présentant une arête tranchante (31) perpendiculaire à la paroi du tube (1),
- un dispositif d'actionnement du couteau (3), comprenant un élément d'actionnement (22, 41) connecté à la poignée de préhension (2) de manière à donner au couteau (3), par rapport au tube (1), un mouvement hélicoïdal dans la direction d'une extrémité distale du tube (1) puis un mouvement de rotation pour détacher l'échantillon du tissu par découpe de sa face située dans la profondeur du tissu.

2. Dispositif selon la revendication 1, dans lequel l'élément d'actionnement est en outre agencé de sorte à, après ledit mouvement de rotation et sous l'effet d'une pression exercée dans la direction de l'extrémité distale (10), faire coulisser le couteau (3) dans le tube (1) de sorte à le faire dépasser de l'extrémité distale (10) du tube (1) en vue de libérer l'échantillon prélevé ;

3. Dispositif selon l'une des revendications 1 ou 2, dans lequel ledit dispositif d'actionnement comprend :
- un élément de connexion (20) solidaire du tube (1),
- une tige d'actionnement (32) solidaire, à son extrémité distale, du couteau (3),
- un élément de retenue (42) solidaire de la tige d'actionnement (2), relié à l'élément de connexion (20) par une liaison hélicoïdale dans une position proximale de la tige d'actionnement (32) par rapport au tube (1) et par une liaison pivot dans une position distale de la tige d'actionnement (32).

4. Dispositif selon la revendication 3, dans lequel ledit élément de connexion (20) comprend une portion proximale (20e) taraudée coopérant avec une collerette filetée (42b) de l'élément de retenue (42) pour former ladite liaison hélicoïdale et un alésage distal (20f) plus large que ladite collerette (42b) pour former ladite liaison pivot.

5. Dispositif selon l'une des revendications 3 ou 4, dans lequel ledit dispositif d'actionnement comprend une molette (41) solidaire de l'extrémité distale de la tige d'actionnement (32).

6. Dispositif selon l'une des revendications 3 à 5, dans lequel l'élément de retenue (42) est maintenu dans l'élément de connexion (20) par une bague (43), ladite bague (43) comprenant une butée (47) proximale pour l'élément de retenue (42) vis-à-vis de l'élément de connexion (20), ladite butée (47) étant escamotable pour libérer l'élément de retenue (42) de l'élément de connexion (20).

7. Dispositif selon l'une des revendications 1 à 6, dans lequel le diamètre intérieur du tube (1) est compris entre 2 et 5 mm.

8. Dispositif selon l'une des revendications 1 à 7, configuré de telle sorte que la course longitudinale du couteau (3) dans son mouvement hélicoïdal est comprise entre 1 et 5 mm.

9. Dispositif selon l'une des revendications 1 à 8, dans lequel la poignée de préhension (2) a une forme générale de pistolet, l'élément d'actionnement comprenant une gâchette (22).

10. Dispositif selon la revendication 9, dans lequel la poignée comprend en outre un bouton de sécurité (23) mobile entre une position de blocage et une position de libération de la gâchette.

## Patentansprüche

1. Vorrichtung zur laparoskopischen Entnahme einer oberflächlichen zylindrischen Probe eines menschlichen oder tierischen Gewebes, umfassend:
- eine Röhre (1), deren Innendurchmesser den Durchmesser der zu entnehmenden Probe bestimmt und aufweisend eine schneidende Kante (12) an ihrem distalen Ende (10), wobei die genannte Kante geeignet ist, die peripherische Wand der Probe auszuschneiden, wenn die Röhre (1) in das Gewebe eindringt;
- einen Griff zum Greifen (2), der fest mit der Röhre (1) in dem Bereich eines proximalen Endes (11) der genannten Röhre (1) verbunden ist,
- ein Messer (3), das im Innern der Röhre (1) angeordnet ist und eine zur Wand der Röhre (1) lotrechte Kante (31) aufweist,
- eine Betätigungsvorrichtung des Messers (3), umfassend ein Betätigungselement (22, 41), das an den Griff zum Greifen (2) derart angeschlossen ist, dass dem Messer (3) im Verhältnis zur Röhre (1) eine schneckenförmige Bewegung in der Richtung eines distalen Endes der Röhre (1), dann eine Rotationsbewegung zur Loslösung der Probe des Gewebes per Ausschneiden ihrer sich in der Tiefe des Gewebes befindenden Seite verliehen wird.

2. Vorrichtung gemäß Anspruch 1, bei der das Betätigungselement darüber hinaus derart angeordnet ist, dass nach der genannten Rotationsbewegung und unter der Wirkung eines in der Richtung des distalen Endes (10) ausgeübten Drucks das Messer (3) in der Röhre (1) derart zum Gleiten gebracht wird, dass das distale Ende (10) der Röhre (1) überschritten wird, um die entnommene Probe freizusetzen.

3. Vorrichtung gemäß einem der Ansprüche 1 oder 2, bei der die genannte Betätigungsvorrichtung umfasst:
- ein fest mit der Röhre (1) verbundenes Anschlusselement (20),
- einen Betätigungsstift (32), der an seinem distalen Ende fest mit dem Messer (3) verbunden ist,
- ein Rückhalteelement (42), das fest mit dem Betätigungsstift (2) verbunden ist, der durch eine schneckenförmige Verbindung in einer proximalen Position des Betätigungsstiftes (32) im Verhältnis zur Röhre (1) und durch eine Schwenkverbindung in einer distalen Position des Betätigungsstiftes (32) mit dem Anschlusselement (20) verbunden ist

4. Vorrichtung gemäß Anspruch 3, bei der das genannte Anschlusselement (20) einen proximalen Abschnitt (20e) mit Innengewinde umfasst, der mit einem Gewindeflansch (42b) mit Außengewinde des Rückhalteelements (42), um die genannte schneckenförmige Verbindung zu bilden, und mit einer distalen Bohrung (20f), die größer ist als der genannte Flansch (42b), zusammenwirkt, um die genannte Schwenkverbindung zu bilden.

5. Vorrichtung gemäß einem der Ansprüche 3 oder 4, bei dem die genannte Betätigungsvorrichtung ein fest mit dem distalen Ende des Betätigungsstiftes (32) verbundenes Rädchen (41) umfasst.

6. Vorrichtung gemäß einem der Ansprüche 3 bis 5, bei der das Rückhaltelement (42) in dem Anschlusselement (20) durch einen Ring (43) festgehalten wird, wobei der genannte Ring (43) einen proximalen Anschlag (47) für das Rückhalteelement (42) gegenüber dem Anschlusselement (20) umfasst, wobei der genannte Anschlag (47) versenkbar ist, um das Rückhalteelement (42) des Anschlusselements (20) freizusetzen.

7. Vorrichtung gemäß einem der Ansprüche 1 bis 6, bei der der Innendurchmesser der Röhre (1) zwischen 2 und 5 mm inbegriffen ist.

8. Vorrichtung gemäß einem der Ansprüche 1 bis 7, die derartig konfiguriert ist, dass der längsseitige Verlauf des Messers (3) in seiner schneckenförmigen Bewegung zwischen 1 und 5 mm inbegriffen ist.

9. Vorrichtung gemäß einem der Ansprüche 1 bis 8, bei der der Griff zum Greifen (2) eine allgemeine Pistolenform hat, wobei das Betätigungselement einen Abzug (22) umfasst.

10. Vorrichtung gemäß Anspruch 9, bei der der Griff darüber hinaus einen zwischen einer Feststellposition und einer Freisetzungsposition des Abzugs mobilen Sicherheitsknopf (23) umfasst.

## Claims

1. A device for laparoscopically collecting a cylindrical surface sample of a human or animal body tissue, comprising:
- a tube (1), the inner diameter whereof determines the diameter of the sample to be collected, and exhibiting a cutting edge (12) at its distal end (10),
- a gripping handle (2), fastened to the tube (1) in the region of a proximal end (11) of said tube (1),
- a knife (3) positioned inside the tube (1) and having a cutting edge (31),
- a device for actuating the knife (3), including an actuating element (22, 41) connected to the gripping handle (2) so as to give the knife (3) a helical motion in the direction of a distal end of the tube (1), then a rotary motion for detaching the sample from the tissue.

2. The device according to Claim 1, wherein the actuating element is arranged so that, after said rotary motion and under the influence of pressure exerted toward the distal end (10), it causes the knife (3) to slide inside the tube (1) so as to make it protrude from the distal end (10) of the tube (1) for the purpose of releasing the collected sample.

3. The device according to one of Claims 1 or 2, wherein said actuating device comprises:
- a connecting element (20) fastened to the tube (1),
- an actuating rod (32) fastened, at its distal end, to the knife (3),
- a retaining element (42) fastened to the actuating rod (2), connected to the connecting element (20) by a helical connection in a proximal position of the actuating rod (32) with respect to the tube (1) and by a pivoting connection in a distal position of the actuating rod (32).

4. The device according to Claim 3, wherein said connecting element (20) comprises a tapped proximal portion (20e) cooperating with a threaded collar (42b) of the retaining element (42) to form said helical connection and a distal bore (20f) larger than said collar (42b) to form said pivoting connection.

5. The device according to one of Claims 3 or 4, wherein said actuating device comprises a thumbwheel (41) fastened to the distal end of the actuating rod (32) .

6. The device according to one of Claims 3 through 5, wherein the retaining element (42) is held in the connecting element (20) by a ring (43), said ring (43) comprising a proximal stop (47) for the retaining element (42) with respect to the connecting element (20), said stop (47) being retractable to release the retaining element (42) from the connecting element (20).

7. The device according to one of Claims 1 through 6, wherein the inner diameter of the tube (1) is comprised between 2 and 5 mm.

8. The device according to one of Claims 1 through 7, configured in such a way that the longitudinal travel of the knife (3) in its helical motion is comprised between 1 and 5 mm.

9. The device according to one of Claims 1 through 8, wherein the gripping handle (2) has the general shape of a pistol, the actuating element including a trigger (22).

10. The device according to Claim 9, wherein the handle also comprises a safety button (23) movable between a locking position and a position freeing the trigger.
